# EUROPÄISCHE PATENTSCHRIFT

(11) **EP 3 516 067 B1**
(45) Veröffentlichungstag und Bekanntmachung des Hinweises auf die Patenterteilung: **06.04.2022**
(21) Anmeldenummer: 17762078.8
(22) Anmeldetag: 28.08.2017
(51) Int. Cl.: C07H 5/06, C12Q 1/04, C12Q 1/34, A61K 49/00

(54) **VERBINDUNG UND KAUGUMMI UMFASSEND DIE VERBINDUNG ZUM GESCHMACKLICHEN NACHWEIS VON VIREN**
COMPOUND AND CHEWING GUM INCLUDING THE COMPOUND FOR DETECTION OF VIRUSES BY TASTE
COMPOSÉ ET GOMME À MÂCHER CONTENANT LE COMPOSÉ POUR LA DÉTECTION DE VIRUS PAR GOÛT

(30) Priorität: 19.09.2016 DE 102016011298
(43) Veröffentlichungstag der Anmeldung: 31.07.2019
(73) Patentinhaber: Julius-Maximilians-Universität Würzburg, 97070 Würzburg (DE)
(72) Erfinder: MEINEL, Lorenz, 97082 Wuerzburg (DE); MIESLER, Tobias, 97076 Wuerzburg (DE); SEIBEL, Juergen, 97070 Wuerzburg (DE)
(74) Vertreter: Lederer & Keller Patentanwälte Partnerschaft mbB
(86) Internationale Anmeldenummer: PCT/EP2017/071572
(87) Internationale Veröffentlichungsnummer: WO 2018/050429

(56) Entgegenhaltungen:
- WO-A1-00/37942
- WO-A1-2013/132058
- US-A- 5 252 458
- US-A- 5 719 020
- THE EUROPEAN COMMISSION: "Commission implementing regulation (EU) No 872/2012", OFFICIAL JOURNAL OF THE EUROPEAN UNION, Bd. L267/1, 2. Oktober 2012 (2012-10-02), Seiten 1-161, XP055413826,
- EFSA: "Scientific Opinion on the safety and efficacy of phenol derivatives containing ring-alkyl, ring-alkoxy and side-chains with an oxygenated functional group (chemical group 25) when used as flavourings for all species : Phenol derivatives (CG 25)", THE EFSA JOURNAL, Bd. 10, Nr. 2, 1. Februar 2012 (2012-02-01), Seite 2573, XP055414332, Parma, IT ISSN: 1831-4732, DOI: 10.2903/j.efsa.2012.2573
- HAGHANI AMIN ET AL: "In vitroandin vivomechanism of immunomodulatory and antiviral activity of Edible Bird's Nest (EBN) against influenza A virus (IAV) infection", JOURNAL OF ETHNOPHARMACOLOGY, ELSEVIER IRELAND LTD, IE, Bd. 185, 11. März 2016 (2016-03-11), Seiten 327-340, XP029527374, ISSN: 0378-8741, DOI: 10.1016/J.JEP.2016.03.020

## Beschreibung

Virale Infektionen lösen eine Vielzahl von Erkrankungen aus, welche weite Teile der Bevölkerung und das Gesundheitssystem jedes Jahr im erheblichen Umfang belasten. Vor allem Infektionen mit dem Influenza A und B Virus betreffen hierbei die Allgemeinheit im großen Maßstab. Laut Robert Koch-Institut erkranken allein in Deutschland jedes Jahr zwei bis zehn Millionen Menschen an der Grippe, wobei die Todesfälle im Jahre 2013 mit 20.600 geschätzt werden (Robert Koch-Institut, Epidemiologisches Bulletin Nr. 3 vom 19. Januar 2015). Oft sind die Mortalitäts-Raten aber noch höher, da viele Personen nicht direkt an der Infektion versterben, sondern an einer ungenügenden Ausheilung sowie der Schwächung des Immunsystems. Durch die unspezifischen Symptome, wie Fieber, Kopf- und Gliederschmerzen, Schnupfen, Husten, Übelkeit oder Erbrechen, ist es sehr oft schwierig, diese Erkrankung korrekt zu diagnostizieren. Zudem tragen häufige Mutationen der Virusstämme dazu bei, dass sich Virulenz und Stärke der Symptome sehr häufig und schnell verändern.

Eine verlässliche Möglichkeit der Diagnose trägt dazu bei, schnell und spezifisch die Infektion mit dem Virus nachzuweisen. Hierzu werden oft Antikörper-basierte Immunassays herangezogen, welche bestimmte Epitope der Viren durch spezifische Bindung identifizieren. Außerdem steht eine PCR-basierte Methode zur Verfügung, mit der charakteristische Abschnitte im Erbgut des Virus nachgewiesen werden können. Beide Methoden haben den Nachteil, sehr teuer, zeitaufwendig und im Umgang kompliziert zu sein, so dass sie lediglich eine geringe Breitenwirkung haben. Zudem haben Immunassays den Nachteil, dass die bei Influenza-Viren durch häufige Mutationen teilweise veränderten Oberflächen von den Assays nicht mehr erkannt werden.

Für Viren spezifische, enzymatisch aktive Proteine - diese befinden sich auf deren Hülle - welche essentiell für ihre Replikation sind, können ebenfalls genutzt werden, um einen Virusnachweis durchzuführen. So exprimiert das Influenza-Virus eine virusspezifische Neuraminidase, welche nach außen abgegeben wird.

In der Natur liegt N-Acetylneuraminsäure (Neu5Ac) [1] (hier dargestellt mit nummerierten C-Atomen) in der Glykokalix von höheren Lebewesen endständig gekuppelt an Glykanketten vor und wird von Neuraminidase im Prozess der VirusFreisetzung von diesen abgespalten.

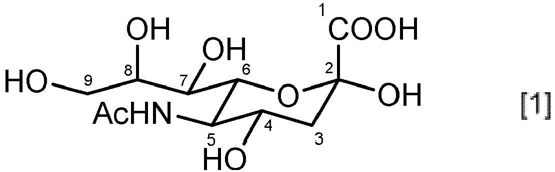

Position 2 von [1] ist hier das anomere Kohlenstoffatom, an welchem die glykosidische Bindung zu den Zuckerketten sitzt. Diese Verknüpfung wird von Neuraminidase im Verlauf der Virusfreisetzung hydrolytisch gespalten. Es ist also logisch an dieser Stelle eine Verbindung anzubringen, welche nach Abspaltung detektiert werden kann.

Eine Methode wie der Nachweis der Anwesenheit von Influenza-Viren mittels der Reaktion dieses Enzyms erfolgen kann wird in US. Pat. No. 5,252,458 beschrieben. Um eine selektive Detektion von nur viral bedingten Erkrankungen zu erreichen, wird die eingesetzte N-Acetylneuraminsäure leicht modifiziert. In Sialinsäuren spielt die Position 4 eine wichtige Rolle bei der Interaktion zwischen Enzym und Substrat. Es wurde gezeigt, dass 4-Methoxy-Neu5Ac von bakteriellen Sialidasen überhaupt nicht, von viralen Sialidasen hingegen sehr schnell geschnitten wird (Beau et al., Eur. J. Biochem., 106 (1980) 531-540). Diese Differenzierung wird durch eine Alkylierung der Hydroxy-Gruppe an Position 7 zusätzlich verstärkt, so dass auch zwischen unterschiedlichen Virus-Neuraminidasen unterschieden werden kann. In US. Pat. No. 5,719,020 wird gezeigt, dass die 4,7-modifizierte Neu5Ac angebunden an eine chromogene Komponente zwischen Influenza A und B differenzieren, sowie gleichzeitig bakterielle von viralen Neuraminidasen unterscheiden kann.

Ein ähnlicher Sensor, welcher bei Kontakt mit Neuraminsäure einen detektierbaren Farbstoff freisetzt, wurde bereits zur Marktreife gebracht (ZymeTx, ZstatFlu^{™} Test for Influenza Types A and B). Nachteilig an diesem System ist allerdings die komplizierte Handhabung, welche ebenso wie die anderen genannten Verfahren von Fachärzten durchgeführt werden muss. WO2013/132058 beschreibt ein diagnostisches Kaugummi zur Detektion oral nachweisbarer Krankheitserreger mittels proteolytischer Spaltung von Substraten, die an maskierte Geschmacksstoffe gekoppelt sind.

Aufgabe der vorliegenden Erfindung ist somit die Bereitstellung eines Mittels bzw. eines Verfahrens, welches dem Patienten ermöglicht, eine viralen Infektion selbst festzustellen, und welches dem Arzt ermöglicht, eine virale Infektion in einfacher Weise von einer bakteriellen Infektion zu unterscheiden und so den unnötigen Einsatz von Antibiotika zu verhindern.

Diese Aufgabe wird erfindungsgemäß gelöst durch eine Verbindung der folgenden allgemeinen Formel [A zur Verwendung in einer Methode zur in vivo-Diagnose einer viralen Infektion 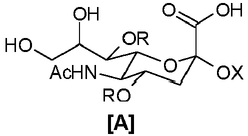 wobei die Reste R unabhängig voneinander Wasserstoff oder C1-C6 Alkyl, vorzugsweise Methyl, darstellen, und sich der Rest -OX von einem Aromastoff oder einem Farbstoff mit der allgemeinen Formel HOX ableitet, wobei es sich bei OH um eine Hydroxygruppe und bei X um einen aliphatischen, aromatischen oder heterozyklischen Rest handelt, wobei es sich bei dem Aromastoff um Thymol oder Carvacrol handelt.

Die Aufgabe wird ferner gelöst durch ein Kaugummi zur Verwendung in einer Methode zur in vivo-Diagnose einer viralen Infektion, umfassend die erfindungsgemäße Verbindung.

Ausgehend von dem ZstatFlu^{™} Test haben wir nun die optische Anwendung dieses Systems ausgeweitet, indem nunmehr durch eine Geschmacks- bzw. Geruchssensation die Erkennung erfolgen kann. Der Nachweis wird also nicht außerhalb des menschlichen Körpers durchgeführt, sondern direkt in der Mundhöhle durch Verwendung der menschlichen Zunge (Geschmackssinn) und der Nase (Geruchssinn) als Detektor, wobei der Nachweis insbesondere durch den Geschmackssinn erfolgt. Kommt die synthetisierte Verbindung in Kontakt mit der Neuraminidase eines dieser Virustypen wird ein Aromastoff freigesetzt, welcher nun gustatorisch bzw. olfaktorisch wahrgenommen werden kann.

Der erfindungsgemäße Verbindung wird in einem Kaugummi formuliert und kommt somit ausreichend lange mit dem Neuraminidase enthaltenden Speichel in Berührung, um reagieren zu können.

Somit kann der Patient leicht selbst diagnostizieren, ob die nicht eindeutigen Symptome wie z.B. "Fieber" von einer bakteriellen oder einer viralen Infektion herrührt. Auch für den Arzt sind solche Informationen bei der nachfolgenden Behandlungsstrategie von Bedeutung. Durch eine gesicherte Diagnose der fälschlicherweise oft mit Antibiotika behandelten Grippe ist es ebenso möglich, die Verordnung von Antibiotika ohne relevanten Nutzen, wirksam zu reduzieren.

Die vorliegende Erfindung betrifft außerdem Verfahren zur Herstellung der Verbindung.

### Zusammenfassung der Erfindung

In der Zusammenfassung betrifft die vorliegende Erfindung:
[1] Verbindung der folgenden allgemeinen Formel [A] zur Verwendung in einer Methode zur in vivo-Diagnose einer viralen Infektion, 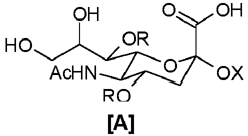 wobei die Reste R unabhängig voneinander Wasserstoff oder C1-C6 Alkyl, vorzugsweise Methyl, darstellen, und sich der Rest -OX von einem Aromastoff mit der allgemeinen Formel HOX ableitet, wobei es sich bei OH um eine Hydroxygruppe und bei X um einen aliphatischen, aromatischen oder heterocyclischen Rest handelt, wobei es sich bei dem Aromastoff um Thymol oder Carvacrol handelt.
[2] Verbindung zur Verwendung gemäß Punkt [1], wobei es sich bei der Verbindung der Formel A um Neu5Ac-Thymol mit der folgenden Formel [5a] 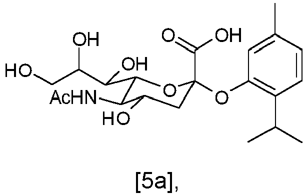 um Neu5Ac-Carvacrol mit der folgenden Formel [5b] 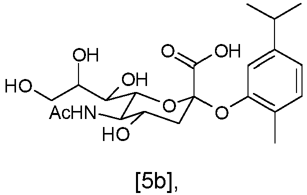 um 4,7-Di-O-methyl-Neu5Ac-Thymol mit der folgenden Formel [12a] 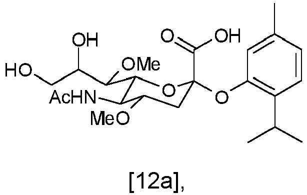 oder um 4,7-Di-O-methyl-Neu5Ac-Carvacrol mit der folgenden Formel [12b] 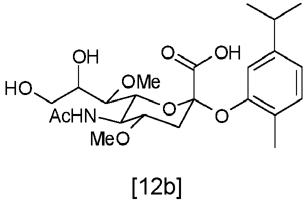 handelt.
[3] Verfahren zur Herstellung einer Verbindung gemäß einem der Punkte [1] und [2], umfassend:
   (a) Verknüpfen von Thymol oder Carvacrol, mit einer Verbindung der folgenden Formel [3] 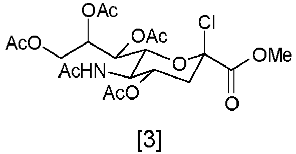 oder einer Verbindung der folgenden Formel [10] 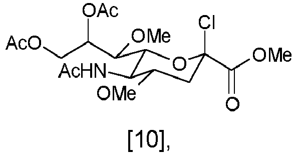
   (b) Entschützen des in Schritt (a) erhaltenen Kupplungsprodukts.
[4] Kaugummi zur Verwendung in einer Methode zur in vivo-Diagnose einer viralen Infektion, umfassend eine Verbindung gemäß einem der Punkte 1 und 2.

### Detaillierte Beschreibung der Erfindung

Bei einem "Aromastoff" handelt es sich um einen Stoff, welcher bei Freisetzung in der Mundhöhle einer Person, vorzugsweise einem Patienten mit einer Virusinfektion, eine geschmackliche (gustatorische) bzw. geruchliche (olfaktorische) Wahrnehmung auslöst. Insbesondere tritt dabei eine geschmackliche Wahrnehmung auf, sodass es sich bei dem Aromastoff insbesondere um einen Geschmacksstoff handelt. Die im Rahmen der vorliegenden Erfindung verwendeten Aromastoffe verfügen über die Eignung, in Lebensmitteln verwendet zu werden.

Bei den Aromastoffen handelt es sich um Aromastoffe mit der allgemeinen Formel HOX, wobei es sich bei OH um eine Hydroxygruppe und bei X um einen aliphatischen, aromatischen oder heterocyclischen Rest handelt.

Bei den Aromastoffen, welche im Rahmen der vorliegenden Erfindung verwendet werden, handelt es sich um Carvacrol und Thymol.

Kupplungsprodukte, die sich von N-Acetylneuraminsäure bzw. einem Derivat davon ableiten, werden im Rahmen der vorliegenden Erfindung der Einfachheit halber als "Neu5Ac-Aromastoff" bzw. "Derivat von Neu5Ac-Aromastoff" bezeichnet. Ein Kupplungsprodukt, das sich also beispielsweise von N-Acetylneuraminsäure (Neu5Ac) und Thymol ableitet, wird im Rahmen der vorliegenden Erfindung als "Neu5Ac-Thymol" bezeichnet.

Die Erfindung zeigt eine Syntheseroute auf, mit welcher N-Acetylneuraminsäure in quantitativer Ausbeute an verschiedene Aromastoffe gekuppelt werden kann. In Kombination mit N-Acetylneuraminsäure bzw. deren 4,7-C1-C6 alkyliertem Derivat kann der Aromastoff nicht bzw. kaum vom Patienten geschmacklich wahrgenommen werden. Kommt das System jedoch in Kontakt mit viraler Neuraminidase, wird die Verbindung gespalten und Neu5Ac (bzw. 4,7-(C1-C6)alkoxy-Neu5Ac) sowie der Aromastoff in seiner alkoholischen OH-Form entstehen. Der freie Aromastoff kann nun wieder gustatorische und olfaktorische Reize in signifikantem Maße auslösen. Eine Wahrnehmung durch den Patienten wird somit möglich, so dass durch ein Auftreten von Geschmack sowie Geruch der gewählten gekuppelten Verbindung, oder durch eine Färbung der Zunge eine durch Influenza-Viren ausgelöste Grippe nachgewiesen wird.

Die Struktur des Systems, welches beispielhaft synthetisiert wurde, kann mit folgender Formel [B] charakterisiert werden: 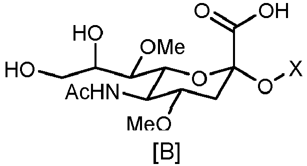 wobei sich -OX von Thymol oder Carvacrol ableitet. Thymol bzw. Carvacrol werden bei Kontakt mit dem Enzym Neuraminidase von 4,7-Dimethoxy-N-acetylneuraminsäure abgespalten und lösen in der freien Form HO-X eine Geschmackssensation bzw. Geruchssensation aus. Das 2-Ketosid ist hierbei an den Aromastoff in der β-anomeren Konfirmation gekuppelt.

Den Verbindungen Thymol und Carvacrol gemeinsam ist ein phenolisches System, welches die deprotonierte Hydroxy-Gruppe ausreichend stabilisiert, die bei der Kupplung zum nukleophilen Angriff an das anomere C2 der Neu5Ac benötigt wird. Durch unsere Synthese-Strategie ist es nunmehr möglich, die genannten Verbindungen an Neu5Ac bzw. deren Derivate anzuknüpfen.

Als Startmaterial diente N-Acetylneuraminsäure, welche von CarboSynth (Compton - Berkshire - UK) bezogen wurde.

Im ersten Schritt der Synthese wird diese in den Methylester überführt. Hierbei wird die Carboxygruppe in Position 1 durch eine Behandlung mit Methanol unter Bedingungen einer sauren Katalyse unter Bildung des Produkts [2] methyliert.

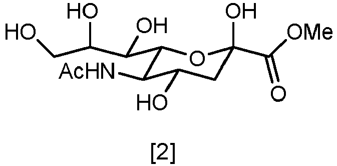

Für diesen Schritt ergeben sich Ausbeuten von 80 - 85 %.

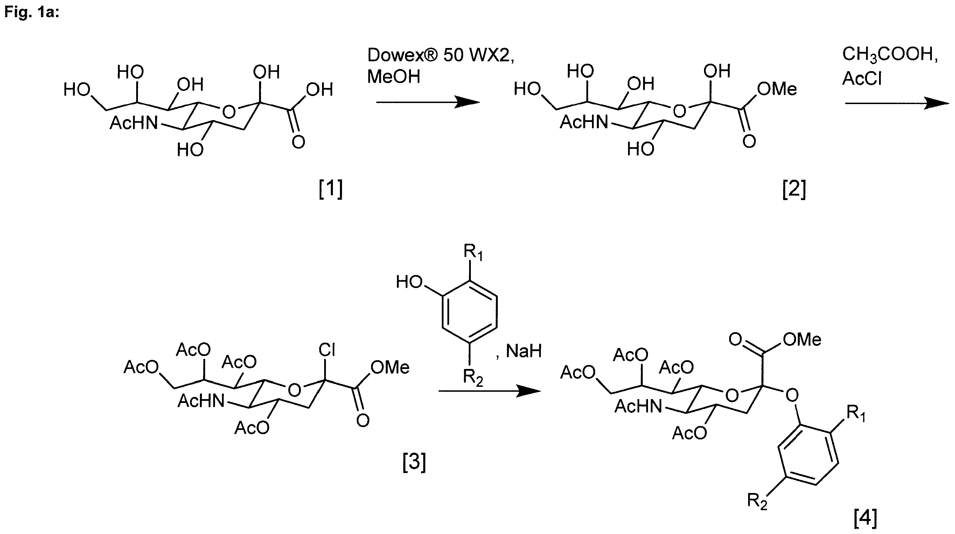

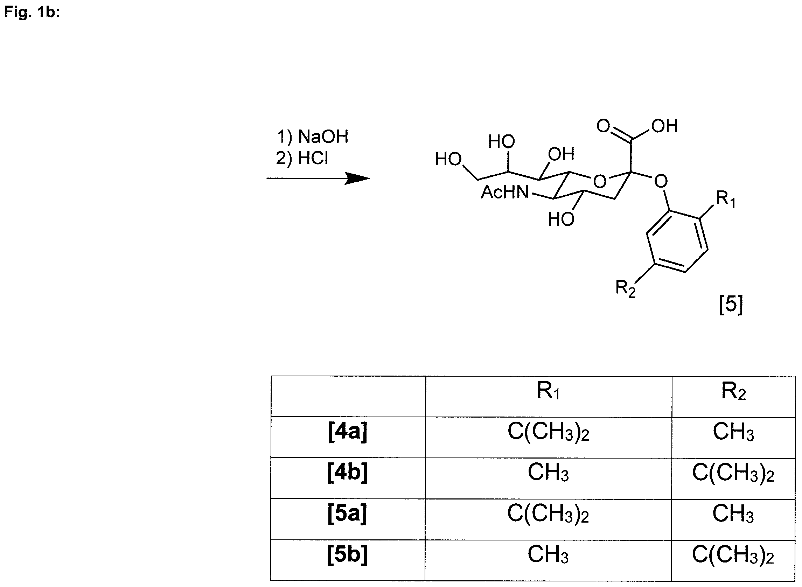

Anschließend wird der Methylester in Essigsäure mit Acetylchlorid (AcCI) behandelt, wodurch einerseits freistehende Hydroxygruppen acetyliert werden, andererseits die Hydroxygruppe in Position 2 durch ihre besondere Reaktivität als Halbacetal in ein Chlorid überführt wird. Aufgrund der Reaktivität und Instabilität wird mit dem erhaltenen Produkt [3] dieser Reaktion unmittelbar weitergearbeitet. 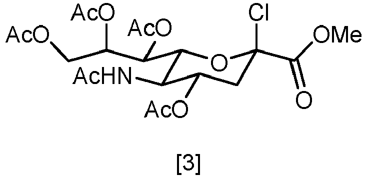 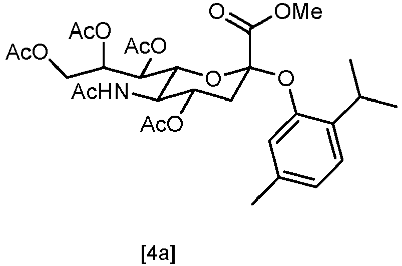 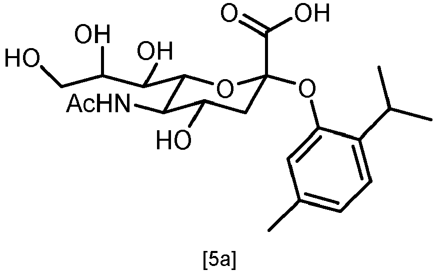 Mittels Natriumhydrid (NaH) wird nun die zu kuppelnde Verbindung deprotoniert, so dass sie am aktivierten C2 der Verbindung nukleophil angreifen kann. Unter Ausschluss von Luftsauerstoff (mittels Schutzgas N₂) kann nun die Kupplungsreaktion von N-Acetylneuraminsäure und dem gewählten Aromastoff ablaufen. Unter den Bedingungen ergaben sich bei Kupplung mit Thymol Ausbeuten von ca. 30 %. Hierbei wird folgendes Produkt [4a] gebildet: Nach Abspaltung der Schutzgruppen in den Positionen 4,7,8,9 durch basische Behandlung der Verbindung, z.B. durch wässrige NaOH, sowie nach Verseifung des Methylesters ergibt sich folgendes Endprodukt [5a]: Im vorliegenden Fall wurde Neu5Ac mit Thymol gekuppelt. Das ebenfalls gut geschmacklich wahrnehmbare Isomer des Thymols Carvacrol, bei welchem die Isopropyliden-Gruppe und die Methyl-Gruppe im Vergleich zum Thymol vertauscht sind ist eine weitere mögliche Kupplungskomponente. Da bei diesem die sterische Hinderung geringer, aber die Reaktivität ansonsten vergleichbar ist, kann diese Verbindung mit dem bei Thymol verwendeten Synthese-Schema ebenso gekuppelt werden. Die Synthesestrategie von Neu5Ac-Thymol und Neu5Ac-Carvacrol wird in Fig. 1 zusammengefasst.

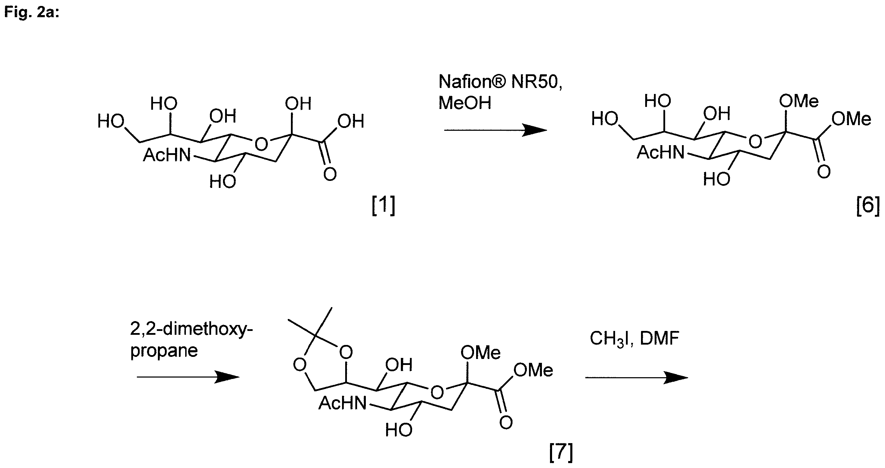

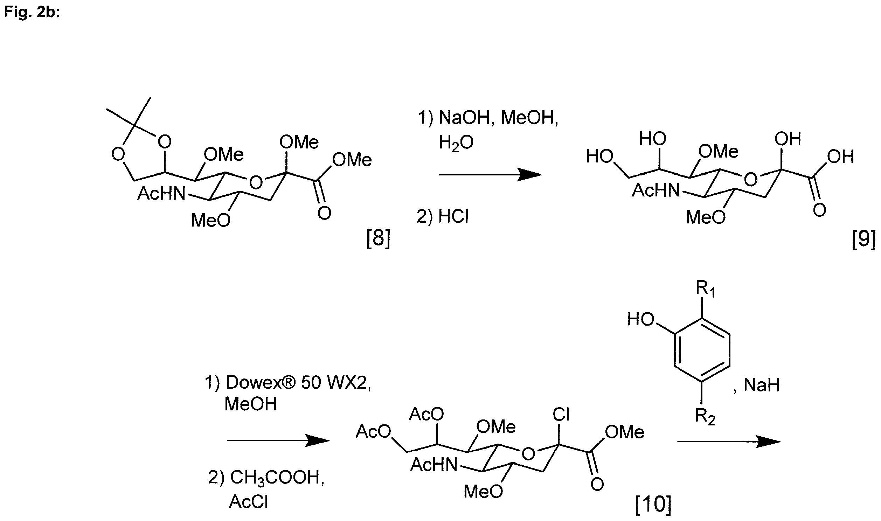

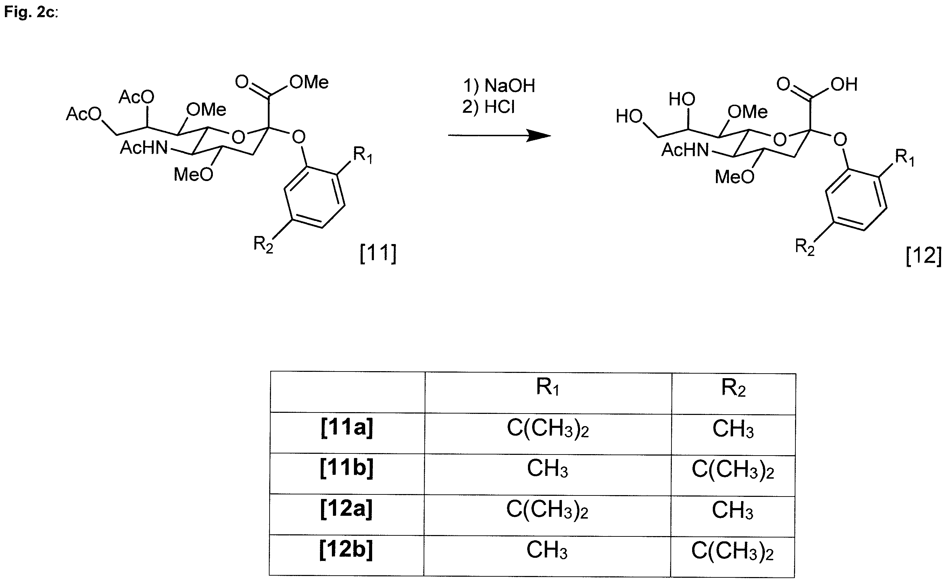

Bei der Kupplung des Aromastoffs mit dem 4,7-Di-O-methyliertem Derivat müssen zuerst die Methylgruppen an der Neu5Ac eingeführt werden, bevor die Verknüpfung beider Komponenten passieren kann. Die Synthese einer kuppelbaren Verbindung wird ausführlich in US. Pat. No. 6,303,764 B1 behandelt und wird wie dort beschrieben durchgeführt. Nur der letzte Schritt, die Kupplung mit der nun 4,7-Di-O-methylierten Neu5Ac wird abgeändert, so dass unsere Verbindungen gekuppelt werden können. Die Synthese-Strategie wird in Fig. 2 schematisch aufgezeigt, wobei die in den Veröffentlichungen beschriebene Komponente [10] mit den von uns verwendeten Aromastoffen verknüpft wird. In früheren Publikationen und Patenten wurden die unveränderte, sowie einfach- und dimethylierte Neu5Ac an zahlreiche fluorogene und chromogene Komponenten gekuppelt. Zwischen den verschiedenen Neu5Ac-Derivaten gab es keine signifikanten Unterschiede, was die Reaktivität bei der Kupplung betrifft. Um eine selektive Kupplung zu erreichen, wird die Koenigs-Knorr-Methode auf die dimethylierte Verbindung angewandt. Hierbei wird mittels Silbercarbonat die phenolische Gruppe des Aromastoffs an das Neu5Ac-Derivat gekuppelt. Hierbei wird wie bei der unmethylierten Variante durch Reaktion mit Acetylchlorid zuerst ein Glycosylchlorid [10] gebildet, wobei die restlichen HydroxyGruppen gleichzeitig wieder mit Acetyl-Schutzgruppen versehen werden. Durch Reaktion des Glycosylchlorids mit Silbercarbonat bildet sich final ein Dioxolaniumion, welches durch eine alkoholische Gruppe angegriffen werden kann. Hierbei wird folgendes Produkt [11] gebildet:

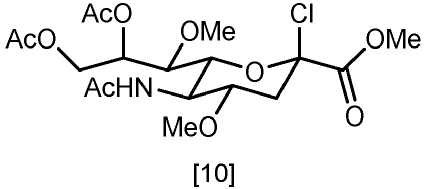

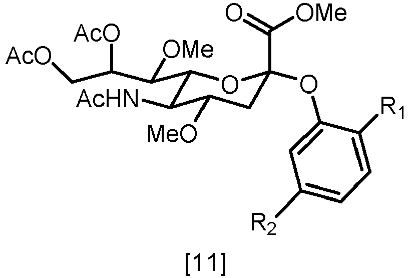

Durch Entschützung mit katalytischen Mengen Natriummethanolat in Methanol ergibt sich das ungeschützte Endprodukt [12]: 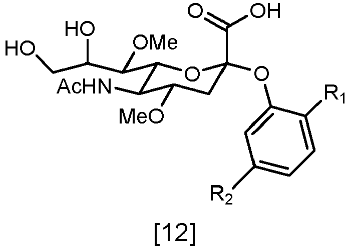 Anwendungsbeispiele sind nicht nur die Verbindungen Neu5Ac-Thymol [5a] sowie Neu5Ac-Carvacrol [5b], sondern zudem die davon abgeleiteten Verbindungen 4,7-Di-O-methyl-Neu5Ac-Thymol [12a] sowie 4,7-Di-O-methyl-Neu5Ac-Carvacrol [12b].

Die Erfindung wird durch die folgenden Beispiele veranschaulicht.

### Beispiel 1: Synthese von Neu5Ac-methylester [2]

1,563 g Neu5Ac [1] (CarboSynth) wird in 50 ml wasserfreiem Methanol dispergiert. Es wird 2 g Dowex 50 W X 2 (gewaschen mit trockenem Methanol) hinzugegeben und der Ansatz bei Raumtemperatur 5 Stunden gerührt. Nach dieser Zeit wird der Ionenaustauscher abfiltriert und mehrmals mit Methanol gewaschen. Nach Entfernen des Methanols durch Abdestillation wird das Produkt [2] mittels Kieselgel aufgereinigt (Fließmittel: Methanol) um 1,354 g (83 %) [2] zu erhalten.

### Beispiel 2: Synthese von Neu5Ac-Thymol [5a]

Zu einer Lösung des Methylesters von Neu5Ac [2] (603 mg, 1,95 mmol) in Essigsäure (10 ml) wird Acetylchlorid (10 ml) gegeben und der Ansatz verschlossen bei Raumtemperatur für 18 h unter Rühren stehen gelassen. Anschließend wird die Lösung unter Vakuum eingeengt und mittels Toluen-Zugabe getrocknet um [3] als weißen Rückstand zu erhalten. Das Produkt wird aufgrund der Instabilität des Chlorids ohne weitere Aufreinigung verwendet.

Zu einer Suspension von NaH (60 % in Mineralöl, 82 mg, 3,429 mmol) in Tetrahydrofuran (THF) wird eine Lösung von Thymol (510 mg, 3,40 mmol) in Dimethylformamid (DMF) (10 ml) bei 0 °C langsam hinzugetropft und das Reaktionsgemisch 10 min gerührt. Die Mischung wird innerhalb von 15 Minuten mit der Lösung des Chlorids [3] in THF versetzt. Nach der vollständigen Zugabe wird das Gemisch 19 Stunden bei Raumtemperatur gerührt. Nach Ablauf der Zeit wird das Gemisch mit 40 ml Ethylacetat verdünnt und in einen Scheidetrichter überführt. Die organische Phase wird nun mit Wasser (2*30 ml) und mit gesättigter Natriumchlorid-Lösung (2*30 ml) gewaschen, über Na₂SO₄ getrocknet und das Lösemittel unter reduziertem Druck entfernt.

Der Rückstand (enthaltend [4a]) wird in Methanol gelöst (8 ml), mit 0,1 _{M} NaOH (2 ml) versetzt und für eine Stunde bei Raumtemperatur gerührt. Der Reaktionsansatz wird mit 3 _{M} HCl auf pH = 4,5-5 gebracht und mit Dichlormethan (DCM) extrahiert (3 * 15 ml). Die Wasserphase wird mit einem FPLC System (Äkta purifier, GE Healthcare) unter Verwendung von Reversed Phase-Chromatographie (RPC) an einer C18- Säule (Phenomenex^{®} aufgereinigt (Eluent A: 0,1 % Trifluoressigsäure (TFA) in Wasser, Eluent B: 0,1 % TFA in Acetonitril). Nach anschließender Gefriertrocknung erhält man N-Acetylneuraminsäure-Thymol [5a] als weißes Pulver. Die Gesamtausbeute beträgt 253 mg (29,4 %).

### Beispiel 3: Synthese von 4,7-Di-O-methyl-Neu5Ac-Thymol [12a]

Zu einer Lösung des Methylesters von 4,7-Di-O-methyl-Neu5Ac [9a] (33 mg, 0,094 mmol) in Essigsäure (3 ml) wird Acetylchlorid (3 ml) gegeben und der Ansatz verschlossen bei Raumtemperatur für 18 h unter Rühren stehen gelassen. Anschließend wird die Lösung unter Vakuum eingeengt und mittels Toluen-Zugabe getrocknet um [3] als weißen Rückstand zu erhalten. Das Produkt wird aufgrund der Instabilität des Chlorids ohne weitere Aufreinigung verwendet.

Das beta-Silylchlorid [10a] wird in 10 ml wasserfreiem DCM gelöst. In einem weiteren Kolben werden unter Licht- und Sauerstoff-Ausschluss 586 mg Thymol (3,9 mmol), 95 mg Silbercarbonat (0,345 mmol) und 300 mg Molekularsieb 4A in 10 ml wasserfreiem DCM. Die erste Lösung mit dem Edukt wird langsam zur zweiten hinzugegeben und für 72 h bei Raumtemperatur gerührt.

Nach Ablauf der Zeit wird das Gemisch mit 40 ml Ethylacetat verdünnt und in einen Scheidetrichter überführt. Die organische Phase wird nun mit Wasser (2*30 ml) und mit gesättigter Natriumchlorid-Lösung (2*30 ml) gewaschen, über Na₂SO₄ getrocknet und das Lösemittel unter reduziertem Druck entfernt. Der Rückstand (enthaltend [11a]) wird in Methanol gelöst (8 ml), mit 0,1 _{M} Natriummethanolat bis auf einen pH von 8-9 versetzt und für eine Stunde bei Raumtemperatur gerührt. Der Reaktionsansatz wird mit 1 _{M} HCl neutralisiert, filtriert und mit Dichlormethan (DCM) extrahiert (3 * 15 ml). Die Wasserphase wird mit einem FPLC System (Äkta purifier, GE Healthcare) unter Verwendung von Reversed Phase-Chromatographie (RPC) an einer C18- Säule (Phenomenex^{®}) aufgereinigt (Eluent A: 0,1 % Trifluoressigsäure (TFA) in Wasser, Eluent B: 0,1 % TFA in Acetonitril). Nach anschließender Gefriertrocknung erhält man 4,7-Di-O-methyl-Neu5Ac-Thymol [12a] als weißes Pulver. Die Gesamtausbeute beträgt 10,2 mg (23,1 %).

## Patentansprüche

1. Verbindung der folgenden allgemeinen Formel [A] zur Verwendung in einer Methode zur in vivo-Diagnose einer viralen Infektion 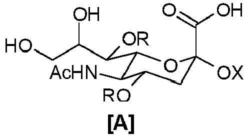 wobei die Reste R unabhängig voneinander Wasserstoff oder C1-C6 Alkyl, vorzugsweise Methyl, darstellen, und sich der Rest -OX von einem Aromastoff mit der allgemeinen Formel HOX ableitet, wobei es sich bei OH um eine Hydroxygruppe und bei X um einen aliphatischen, aromatischen oder heterocyclischen Rest handelt, wobei es sich bei dem Aromastoff um Thymol oder Carvacrol handelt.

2. Verbindung zur Verwendung gemäß Anspruch 1, wobei es sich bei der Verbindung der Formel A um Neu5Ac-Thymol mit der folgenden Formel [5a] 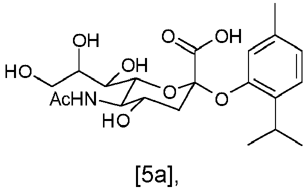 um Neu5Ac-Carvacrol mit der folgenden Formel [5b] 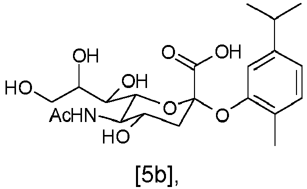 um 4,7-Di-O-methyl-Neu5Ac-Thymol mit der folgenden Formel [12a] 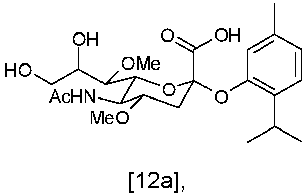 oder um 4,7-Di-O-methyl-Neu5Ac-Carvacrol mit der folgenden Formel [12b] 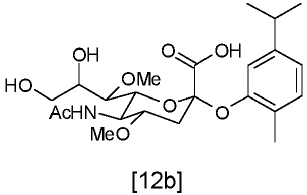 handelt.

3. Verfahren zur Herstellung einer Verbindung gemäß einem der Ansprüche 1 und 2, umfassend:
(a) Verknüpfen von Thymol oder Carvacrol, mit einer Verbindung der folgenden Formel [3] 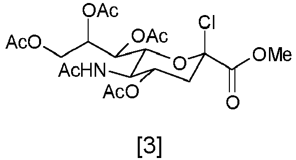 oder einer Verbindung der folgenden Formel [10] 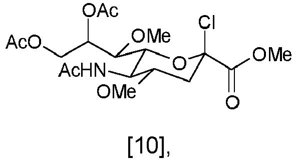 (b) Entschützen des in Schritt (a) erhaltenen Kupplungsprodukts.

4. Kaugummi zur Verwendung in einer Methode zur *in vivo*-Diagnose einer viralen Infektion, umfassend eine Verbindung gemäß einem der Ansprüche 1 und 2.

## Claims

1. A compound of the following general formula [A] for use in a method for the in vivo diagnosis of a viral infection 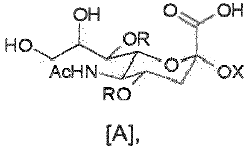 wherein residues R independently represent hydrogen or C1-C6 alkyl, preferably methyl, and residue -OX is derived from a flavoring agent of general formula HOX, wherein OH is a hydroxy group and X is an aliphatic, aromatic, or heterocyclic residue, wherein the flavoring agent is thymol or carvacrol.

2. The compound for use according to claim 1, wherein the compound of formula A is Neu5Ac-thymol of the following formula [5a] 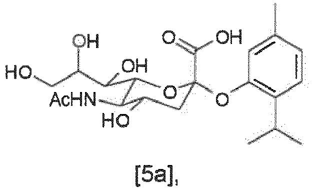 Neu5Ac-carvacrol of the following formula [5b] 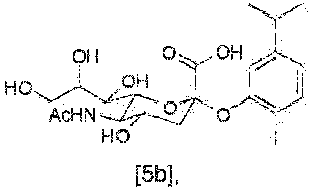 4,7-di-O-methyl-Neu5Ac-thymol of the following formula [12a] 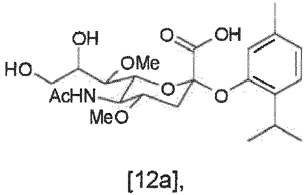 or 4,7-di-0-methyl-Neu5Ac-carvacrol of the following formula [12b]

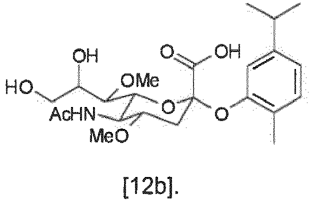

3. A method for the preparation of a compound according to any of claims 1 and 2 comprising:
(a) linking thymol or carvacrol to a compound of the following formula [3] 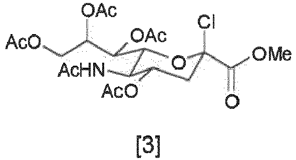 or a compound of the following formula [10] 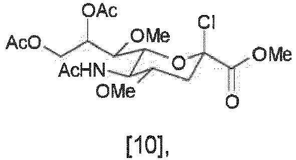
(b) deprotecting the coupling product obtained in step (a).

4. A chewing gum for the use in a method for the in vivo diagnosis of a viral infection comprising a compound according to any of claims 1 and 2.

## Revendications

1. Composé de formule générale [A] ci-dessous, destiné à être utilisé dans un procédé de diagnostic *in vivo* d'une infection virale 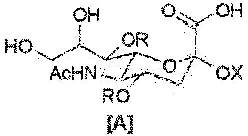 dans lequel les radicaux R représentent indépendamment l'un de l'autre de l'hydrogène ou un alkyle en C1-C6, de préférence du méthyle, et le radical -OX est dérivé d'une substance aromatique de formule générale HOX, dans lequel OH est un groupe hydroxy et X est un radical aliphatique, aromatique ou hétérocyclique, dans lequel la substance aromatique est du thymol ou du carvacrol.

2. Composé destiné à être utilisé selon la revendication 1, dans lequel le composé de formule A est du Neu5Ac-thymol de formule [5a] ci-dessous, 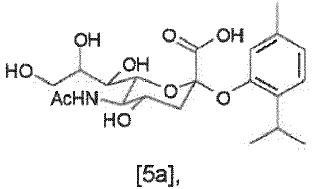 du Neu5Ac-carvacrol de formule ci-dessous [5b], 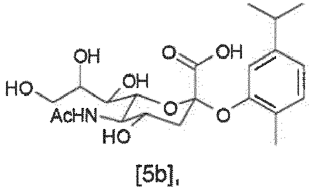 du 4,7-di-O-méthyl-Neu5Ac-thymol de formule [12a] ci-dessous, 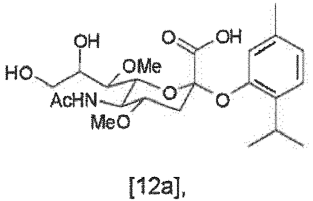 ou du 4,7-di-O-méthyl-Neu5Ac-carvacrol de formule [12b] ci-dessous

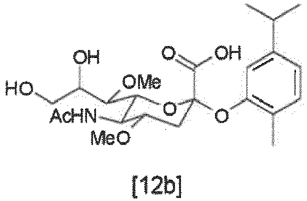

3. Procédé de fabrication d'un composé selon la revendication 1 ou 2, comprenant les étapes consistant à :
(a) lier le thymol ou le carvacrol à un composé de formule [3] ci-dessous 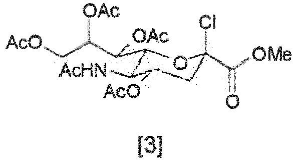 ou à un composé de formule [10] ci-dessous, 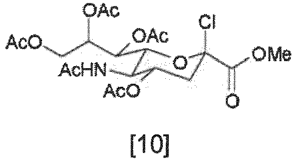
(b) déprotéger le produit de couplage obtenu à l'étape (a).

4. Gomme à mâcher destinée à être utilisée dans un procédé de diagnostic *in vivo* d'une infection virale, comprenant un composé selon la revendication 1 ou 2.
